# EUROPEAN PATENT SPECIFICATION

(11) **EP 2 427 567 B1**
(45) Date of publication and mention of the grant of the patent: **08.04.2015**
(21) Application number: 10718383.2
(22) Date of filing: 04.05.2010
(51) Int. Cl.: C12Q 1/04, C12Q 1/22

(54) **RAPID STERILITY MICROASSAY**
SCHNELLER STERILITÄTSMIKROTEST
MICRODOSAGE RAPIDE MESURANT LA STÉRILITÉ

(30) Priority: 04.05.2009 US 175271 P
(43) Date of publication of application: 14.03.2012
(73) Proprietor: Novartis AG, 4056 Basel (CH)
(72) Inventor: GRAY, Jennifer, Claire, 4125 Riehen (CH); STAERK, Alexandra, 4334 Sisseln (CH); BERCHTOLD, Manfred, 79713 Bad Säckingen (DE)
(74) Representative: Marshall, Cameron John
(86) International application number: PCT/US2010/033503
(87) International publication number: WO 2010/129521

(56) References cited:
- WO-A2-01/59157
- US-A- 4 036 698
- US-A1- 2003 003 540
- WAGNER DEBORAH S ET AL: "Potency and Sterility of Anesthetic Drugs in an Obstetrical Setting" ANESTHESIOLOGY ABSTRACTS OF SCIENTIFIC PAPERS ANNUAL MEETING (2000 ANNUAL MEETING OF THE AMERICAN SOCIETY OF ANESTHESIOLOGISTS; SAN FRANCISCO, CA, USA; OCTOBER 16-18, 2000),, 16 October 2000 (2000-10-16), pages A-1098, XP009135128
- PARVEEN SEEMA ET AL: "Evaluation of growth based rapid microbiological methods for sterility testing of vaccines and other biological products", VACCINE, vol. 29, no. 45, October 2011 (2011-10), pages 8012-8023, ISSN: 0264-410X(print)
- GRAY J C ET AL: "Introduction of a rapid microbiological method as an alternative to the pharmacopoeial method for the sterility test", AMERICAN PHARMACEUTICAL REVIEW 2010 RUSSELL PUBLISHING LLC USA, vol. 13, no. 6, September 2010 (2010-09), pages 88-94, ISSN: 1099-8012

## Description

### BACKGROUND OF THE INVENTION

Federal regulations in the United States, and similar regulations in other nations, require sterility testing to ensure that pharmaceutical products are substantially free of microorganisms such as bacteria and fungi. Three general methods, direct transfer sterility testing, membrane filtration sterility testing and product flush sterility testing, have been used to carry out such sterility testing. The traditional sterility tests are performed with two liquid nutrient media (Tryptic Soy Broth (TSB), incubated at 20-25°C and Thioglycollate Nutrient Medium (FTM), incubated at 30-35°C) and rinsing fluids, and require an incubation time of 14 days. (See, *e.g.,* USP <71> "Sterility Tests," Pharmacopeial Forum. USP 30-NF 25 through First Supplement The United States Pharmacopeial Convention, Inc.), EP 2.1.6 "Sterility" (European Pharmacopoiea 2.1.6 "Sterility" EP 6th Edition (2007)) and in other relevant pharmacopoeias. Fluid Thioglycollate Medium (FTM) contains two phases- the lower phase of the liquid medium is an anaerobic phase, the upper phase contains oxygen for aerobic incubation.

Generally, to test a pharmaceutical product for sterility using the membrane filtration method, a liquid, emulsified or dissolved pharmaceutical or medical product *(e.g*.*,* active components, parenteral formulations, eye drops, nasal spray and the like), is filtered through a 0.45 micron pore size membrane, and the membrane is transferred to appropriate test media (FTM and TSB) and incubated for 14 days. If microbes grow in the cultures, then the pharmaceutical product is not sterile and samples can be taken for microbiological identification. The large amount of time required for incubation, and also for microbiological identification of the organisms that grow in the cultures, is disadvantageous and can limit the availability of pharmaceutical products to patients in need thereof. For example, in times of medical crisis, current sterility tests that require a 14 day incubation period can delay the availability of medicines or vaccines that can reduce or end the crisis. Thus, a need exists for a rapid sterility testing method.

### SUMMARY OF THE INVENTION

The invention relates to a method for sterility testing of a liquid pharmaceutical composition comprising the steps of a) providing a filterable pharmaceutical composition; b) filtering the pharmaceutical composition to provide at least three filter membranes upon which the pharmaceutical composition filtrand is deposited; c) placing the at least three filter membranes onto solid culture media to produce at least three filtrand cultures; d) culturing i) at least one filtrand culture under aerobic conditions at 20 - 25°C; ii) at least one filtrand culture under aerobic conditions at 30 - 35°C; and iii) at least one filtrand culture under anaerobic conditions at 30 - 35°C; with the proviso that none of the filtrand cultures are cultured for a period of more than about 13 days and c) detecting a viable microorganism cell, micro-colony or colony on a membrane, wherein the presence of a viable microorganism cell, micro-colony or colony on the membrane indicates the presence of a viable microorganism in the pharmaceutical composition.

The method can further comprise a step of filtering a wash solution after the pharmaceutical composition is filtered.

In some embodiments, the membrane is a polyvinylidenefluoride membrane, glass fiber membrane, polycarbonate membrane, polyethylene terephthalate membrane, mixed cellulose ester (cellulose acetate and cellulose nitrate), phosphocellulose membrane, DEAE membrane, nylon mesh membrane, or polytetrafluroethylene membrane. Preferably, the membrane has a pore size of about 0.45 µm.

The solid culture media can be selected from the group consisting of FTM-A (fluid thioglycollate medium containing 1.075% agar (final concentration)), BHI (brain heart infusion agar), Difco brewer anaerobic agar, R2A agar, Schaedler blood agar, Caso-agar ICR (tryptic soy agar), Columbia agar 5% blood, and CDC anaerobic blood agar.

In some embodiments, the method can include the step of culturing the filtrand culture for a period of about 2 to about 7 days.

In some embodiments, a viable microorganism cell, micro-colony or colony is detected using a luminescence assay, such as a bioluminescence assay that detects adenosine triphosphate (ATP) produced by a viable microorganism cell, micro-colony or colony on the membrane. The luminescence assay can comprise a luciferase assay.

In some embodiments the luminescence assay detects a nucleic acid hybridization product formed between a probe and a nucleic acid endogenous to a microorganism. The luminescence assay can comprise a peroxidase reaction.

In some embodiments, the luminescence can be detected using a charged coupled device camera and image analysis software. In some embodiments, the number of viable microorganism cells, viable microorganism micro-colonies or viable microorganism colonies can be quantified or enumerated.

In some embodiments, the pharmaceutical composition is a liquid composition. The liquid composition can be a parenteral composition, an oral composition, a nasal composition, an ocular composition, or a vaccine.

The invention also relates to a method for sterility testing of a liquid pharmaceutical composition comprising the steps of a) providing a filterable pharmaceutical composition; b) filtering the pharmaceutical composition to provide at least three filter membranes upon which the pharmaceutical composition filtrand is deposited; c) placing the at least three filter membranes onto solid culture media to produce at least three filtrand cultures; d) culturing i) at least one filtrand culture under aerobic conditions at 20 - 25°C; ii) at least one filtrand culture under acrobic conditions at 30 - 35°C; and iii) at least one filtrand culture under anaerobic conditions at 30 - 35°C; with the proviso that none of the filtrand cultures arc cultured for a period of more than about 13 days; and e) detecting adenosine triphosphate (ATP) on the membrane, wherein the presence of ATP on the membrane indicates the presence of a viable microorganism in the pharmaceutical composition.

### BRIEF DESCRIPTION OF THE DRAWINGS

FIG. 1 is a graph illustrating the results of UV-treatment of *M. osloensis* over a time frame of ten minutes. The number of colony forming units (CFU) present after four days of incubation are shown. The graph shows greater than or equal to 50% reduction of CFU after four minutes (3 runs).
FIG. 2 is a graph illustrating the results of heat-treatment of *E. coli* over a time frame of ten minutes. The number of colony forming units (CFU) after three days of incubation are shown. The graph shows greater than or equal to 50% reduction of CFU after 3 minutes (3 runs).
FIG. 3 is a graph illustrating the results of parenteral drug product treatment over a time frame of ten minutes. The number of colony forming units (CFU) after five days of incubation are shown. The graph shows greater than or equal to 50% reduction of CFU after 2 minutes (3 runs).
FIG. 4A is a photomicrograph of *M. luteus* cells that have been heat treated at 70°C for three minutes. FIG. 4B is a photomicrograph of untreated *M. luteus* cells plated on Tryptic Soy Agar after three days of incubation at 30-35°C.
FIG. 5 is a graph illustrating optical density data for untreated *E. coli* culture, the heat treated *E. coli,* UV-treated *E. coli* and Voltaren-treated *E. coli.*
FIG. 6 is a graph illustrating optical density data for the untreated *E. coli* culture, the heat treated *E. coli,* UV-treated *E. coli* and Voltaren-treated *E. coli.*
FIG. 7 is a graph showing a comparison of untreated *E. coli* growth curves and heat-treated *E. coli.* Over the course of three hours the *E. coli* culture has a slope of 0.2313, then it rises back to the normal slope of the untreated culture.
FIG. 8 is a graph showing a comparison of untreated *S. aureus* growth curves and heat-treated *S. aureus.* Over the course of four hours, the stressed *S. aureus* culture has a slope of 0.1070, then it rises back to the normal slope of the untreated culture (after four hours, 0.4034).
FIG. 9 is a graph showing a comparison of untreated *C. albicans* growth curves and heat-treated *C. albicans.* Over the course of more than eight hours, the stressed *C*. *albicans* culture has a slope of 0.0419.
FIG. 10 is a graph showing a comparison of untreated *B. pumilus* growth curves and starved *B. pumilus*. The cells experienced nutrient depletion for seven days at 2-8°C. Over the course of five hours, the stress *B. pumilus* culture has a slope of -0.0056, then it rises back to the normal slope of the untreated culture.
FIG. 11 is a photomicrograph showing the results of a background test of Schaedler Blood Agar. MILLIFLEX Rapid membranes MXHVWP124 were incubated on Schaedler Blood Agar in MILLIFLEX cassettes for five days at 30-35°C. FIG. 11A shows the MILLIFLEX Rapid image, the membrane was in this case rinsed with 100ml Fluid A. FIG. 11B shows a membrane rinsed with 100ml Fluid D.

### DETAILED DESCRIPTION OF THE INVENTION

The invention relates to a method for sterility testing that is more rapid than conventional tests, which require a 14 day incubation period. The method is particularly well-suited for rapid sterility testing of filterable liquid compositions, such as liquid pharmaceutical compositions *(e.g.,* solutions, suspensions, emulsions, parenteral compositions, oral compositions, nasal compositions, ocular compositions, vaccines). Generally, the method includes filtering a pharmaceutical composition through a filter membrane, the filter membrane is then transferred to a solid culture medium and incubated under appropriate growth conditions for a time sufficient to permit viable microorganisms that are on the membrane to proliferate or produce a sufficient amount of a biomolecule, such as adenosine triphosphate, to allow detection of the microorganism (*e.g.,* 6 hours, 12 hours, 1 day, 2 days, 3 days, 4 days, 5 days, 6 days, 7 days).

In one aspect, the invention is a method for sterility testing of a liquid pharmaceutical composition. The method includes filtering a pharmaceutical composition (*e.g.,* a liquid pharmaceutical composition) through a filter membrane to provide more than one (*e.g.,* at least three) filter membrane upon which the pharmaceutical composition filtrand, and any viable microorganisms that might be in the pharmaceutical composition, are deposited. If desired, a wash solution can then be filtered, for example, to wash growth inhibitors, metabolic inhibitors or detection inhibitors off the membrane, and thereby facilitate detection of microorganisms in the filtrand. The filter membranes containing the filtrand are placed onto solid culture media to produce at least three filtrand cultures. The filtrand cultures are then cultured under three different conditions: aerobic conditions at 20-25°C, aerobic conditions at 30-35°C and anaerobic conditions at 30-35°C for a culture period that is sufficient to permit viable microorganisms that are on the filter membrane to proliferate or produce a sufficient amount of a biomolecule, such as adenosine triphosphate, to allow detection of the microorganism. Generally, the filtrand cultures are cultured for a period of no more than about 13 days, and preferably are cultured for a period that is substantially shorter than 13 days. Then, the filtrand cultures are assessed for the presence of a viable microorganism cell, micro-colony or colony on the membrane, using any suitable method. The presence of a viable microorganism cell, micro-colony or colony on the filter membrane indicates the presence of a viable microorganism in the pharmaceutical composition.

In one aspect of the invention, a method for sterility testing of a liquid pharmaceutical composition is provided, comprising the steps of providing a filterable pharmaceutical composition, filtering the composition to provide at least three filter membranes upon which the filtrand is deposited, placing the three membranes onto solid culture media to produce three filtrand cultures, culturing a first filtrand culture under aerobic conditions at 20-25°C, culturing a second filtrand culture under aerobic conditions at 30-35°C and culturing a third filtrand culture under anaerobic conditions at 30-35°C for a culture period of no more than about 13 days, and detecting adenosine triphosphate (ATP) on the membrane. The presence of ATP on a filter membrane indicates the presence of a viable microorganism in the pharmaceutical composition.

A broad range of microorganisms may be detected using the invention (*e.g.,* yeasts and molds, gram positive sporulating bacteria, gram negative bacteria, gram positive cocci and gram positive rods (both aerobic and anaerobic microorganisms). The method can be used to detect ATCC (American Type Culture Collection) strains, and also for detecting environmental microorganisms that might contaminate manufacturing facilities. For example, as described herein, the method can be used to detect *Aspergillus brasiliensis* ATCC 16404 (formerly known as *Aspergillus niger), Bacillus subtilis* ATCC 6633, *Candida albicans* ATCC 10231, *Clostridium sporogenes* ATCC 11437, *Pseudomonas aeruginosa* ATCC 9027, *Staphylococcus aureus* ATCC 6538, *Escherichia coli* ATCC 8739, *Acinetobacter Iwoffi, Bacillus clausii, Bacillus idriensis, Bacillus licheniformis, Bacillus pumilus, Bacillus sphaericus, Corynebacterium afermentans; Kocuria spez., Kocuria rhizophilia* (formerly known as *Micrococcus luteus*), *Moraxella osloensis, Penicillium spez., Propionibacterium acnes, Staphyloccus capitis, Staphylococcus epidermidis* and *Staphylococcus warneri*.

Generally, the pharmaceutical composition (*e.g.,* liquid pharmaceutical composition) is filtered through a sterile filter membrane using sterile or aseptic technique under pressure, such as using a vacuum or positive pressure. Any suitable filter membrane and filtering device can be used. Examples of suitable filter membrane include, for example, a polyvinylidenefluoride membrane, glass fiber membrane, polycarbonate membrane, polyethylene trephthalate membrane, mixed cellulose ester (cellulose acetate and cellulose nitrate), phosphocellulose membrane, DEAE membrane, nylon mesh membrane or polytetrafluroethylene membrane. Preferably the membrane is made up of PVDF (polyvinylidene fluoride). Filter membrane suitable for use in the claimed invention have a pore size that is sufficiently small to retain microorganisms that might be present in the pharmaceutical composition, such as a pore size of about 0.1 microns (µm) to about 20 microns, about 0.1 microns to about 15 microns, 0.1 microns to about 12 microns, 0.1 microns to about 10 microns, 0.1 microns to about 8 microns, 0.1 microns to about 6 microns, 0.1 microns to about 5 microns, 0.4 microns - 12 microns, 0.4 microns - 10 microns, 0.4 microns - 8 microns, 0.4 microns - 6 microns, about 0.22 microns, or about 0.45 microns. Preferably, the membrane filter has a pore size of about 0.45 microns.

Generally at least three filter membranes that contain filtrand are prepared. This can be accomplished by filtering three separate samples of the pharmaceutical composition through three separate filter membranes. This can also be accomplished by preparing one filter membrane that contains a pharmaceutical composition filtrand and cutting or dividing the filter, using sterile or aseptic technique, into three portions (*e.g.,* three portions of about equal size). The filter membranes that contain the pharmaceutical composition filtrand are then placed onto a suitable solid culture media to produce filtrand cultures.

Suitable solid culture media and culture conditions can be selected that will support growth and/or metabolism of a desired microorganism to be detected. This can be accomplished, for example by screening culture media, as described and exemplified herein. Preferably, the solid culture media used in the method will support growth of a wide range of microorganisms under aerobic and anaerobic culture conditions. If desired, more than one solid culture media can be used. For example, a first culture media can be used that is equivalent to or better than the liquid Tryptic Soy Broth for growth of yeasts, molds and aerobic bacteria; a second solid culture media can be selected that is equivalent to or better than the anaerobic phase of Fluid Thioglycollate Medium for growth of anaerobic microorganisms, and a third solid culture can be selected that is equivalent to or better than the aerobic phase of Fluid Thioglycollate Medium for growth of aerobic microorgansims. Preferably, a single solid culture media is used under both aerobic and anaerobic conditions. Suitable solid culture media that can be used in the invention include, for example, FTM-A (fluid thioglycollate medium containing 1.075% agar (final concentration)), BHI (brain heart infusion agar), Difco Brewer Anaerobic Agar, R2A Agar, Schaedler Blood Agar, Caso-Agar ICR (Tryptic Soy Agar), Columbia Agar 5% Blood or CDC Anaerobic Blood Agar. Schaedler Blood Agar is a particularly preferred solid culture media for use in the method.

Preferred solid culture media are suitable for supporting growth and/or metabolism of "stressed" and "unstressed" microorganisms, as described herein. This is desirable, because microorganisms may have experienced stress, *e.g.,* hypotonic or hypertonic stress, irradiation (such as UV-light, gamma-irradiation, microwaves), ultrasound, heat, low temperature or chemical stress (evoked for example by chlorine or pharmaceutical drug products) during process chemistry, such as during the manufacture of a pharmaceutical composition.

The filtrand cultures are incubated (*i.e.,* cultured) under appropriate growth conditions for a period of time that is sufficient to permit viable microorganisms that are on the membrane to proliferate to produce micro-colonies or colonies, or to produce a sufficient amount of a biomolecule, such as adenosine triphosphate, to allow detection of the microorganism. Generally, one filtrand culture is incubated under aerobic conditions at 30-35°C, a second filtrand culture is incubated under anaerobic conditions at 30-35°C, and a third filtrand culture is incubated under aerobic conditions at 20-25°C.

In some embodiments, the filtrand cultures are cultured for a period of time sufficient to permit viable microorganisms to produce a detectable amount of ATP, such as at least about 200 attomoles of ATP, at least about 200 femtomoles of ATP, at least about 200 picomoles of ATP, or at least about 200 nanomoles of ATP. The culture period may be about 2 to about 7 days, about 2 to about 8 days, about 2 to about 9 days, about 2 to about 10 days, about 2 to about 11 days, about 2 to about 12 days, about 2 to about 13 days, about 6 hours, about 12 hours, about 1 day, about 2 days, about 3 days, about 4 days, about 5 days, or about 6 days. The incubation period for each individual filtrand culture can vary as appropriate, and not all filtrand cultures need to be cultured for the same amount of time. The preferred incubation time will vary based on the microorganism to be detected.

A viable microorganism cell, micro-colony or colony present on a filter membrane after incubation of a filtrand culture may be detected using any suitable method, such as by visual inspection or preferably using a suitable assay. For example, a luminescence assay (*e.g.,* luciferase assay) may be used to detect a viable microorganism cell, micro-colony or colony. Any suitable method or system can be used to detect luminescence, such as a charge coupled device camera, image processor and or image analysis software. Advantageously, image analysis software may be used to quantify or enumerate the number of viable microorganism cells, viable microorganism micro-colonies or viable microorganism colonies. In some embodiments, the luminescence assay, such as a luciferase assay, is used to detect adenosine triphosphate (ATP) that is produced by the viable microorganism cell, micro-colony or colony on the filter membrane. For example, an ATP releasing reagent and a bioluminescent agent (*e.g.,* a reagent that contains luciferase and luciferin) are applied to the filter membranes and luminescence occurs if viable cells produced ATP. Suitable reagents for detecting ATP by luminescence are well-know in the art and are available commercially (*e.g.,* MILLIFLEX rapid reagent kit; Millipore Corporation, Billerica, Massachusetts).

A viable microorganism cell, micro-colony or colony present on a filter membrane after incubation of a filtrand culture can also be detected, for example, using a luminescence assay to detect hybridization of a probe that will hybridize to a nucleic acid endogenous to a microorganism. The luminescence assay may comprise a peroxidase reaction or other suitable reaction. Reagents suitable for producing luminescence through the activity of peroxidase and other enzymes are well known and commercially available.

An assay and detection system may be used that allows about 10-100 yeast cells or about 1000 bacterial cells to be detected. Preferably, an assay and detection system is used that allows a single cell or as few as about 100 cells to be detected. This can be accomplished, for example, by detecting ATP produced by a microorganism. For example, a commercially available ATP bioluminescence assay, when used in combination with a commercially available charged coupled device camera, image processor and image analysis software (MILLIFLEX rapid microbiology detection and enumeration system; Millipore Corporation, Billerica, Massachusetts) can be used to detect about 200 attomoles of ATP, which is equivalent to about 1 yeast or mold cell or about 100 bacterial cells.

The detection method described herein is well-suited for assessing the sterility of filterable compositions, such as liquid pharmaceutical compositions. Liquid compositions include aqueous compositions, suspensions and emulsions. The liquid pharmaceutical composition may be any liquid suitable for pharmaceutical use, including, for example, a parenteral composition, an oral composition, a nasal composition, or an ocular composition. The liquid composition may be a vaccine.

Suitable vaccines include, for example, anthrax vaccine (ANT), Bacile Calmette-Guerin tuberculosis vaccine (BCG), Borreliosis vaccine outer surface protein A vaccine (BORospA), diphtheria toxoid and tetanus toxoid vaccine (DT), diphtheria toxoid and tetanus toxoid and pertussis vaccine (DTP), diphtheria toxoid and tetanus toxoid and acellular pertussis vaccine for peadiatric use(DTPa), diphtheria toxoid and tetanus toxoid and acellular perussis vaccine for adult use (DrTPar), diphtheria toxoid and tetanus toxoid and acellular perussis and Haemophilus influenzae type b conjugate vaccine (DTPa-HIB), diphtheria toxoid and tetanus toxoid and acellular perussis and Haemophilus influenzae type b conjugate and poliovirus inactivated vaccine (DTPa-HIB-IPV), hepatitis A virus vaccine (HAV), hepatitis A virus and hepatitis B virus vaccine (HAV-HBV), hepatitis B virus vaccine (HBV), Helicobacter pylori vaccine (HEL), haemophilus influenzae type b vaccine (HIB), haemophilus influenzae type b conjugate vaccine (HIBcn), haemophilus influenzae type b polysaccharide vaccine (HIBps), haemophilus influenzae type b vaccine (diphtheria CRRM197 protein conjugate, oligosaccharides conjugated to diphtheria CRM197 toxin protein; HIB-HbOC)), influenza virus vaccine (INF) including vaccines for avian influenza (*e.g.,H5N1,* H1N3) and swine influenza (*e.g.,* H1N1), influenza virus attenuated live vaccine (INFa), influenza virus attenuated live vaccine intranasal (INFan), influenza virus inactivated vaccine (INFi), influenza virus inactivated vaccine split virion (INFs), influenza virus inactivated vaccine split virion types A and B trivalent (INFs-AB3), influenza virus vaccine whole virion (INFw), poliovirus inactivated vaccine (IPV), meningococcal (neisseria meningitides) vaccine (MEN), meningococcal (neisseria meningitides) conjugate vaccine (MENcn), meningococcal (neisseria meningitides) conjugate vaccine serogroups A, C (MENcn-AC), meningococcal (neisseria meningitides) polysaccharide vaccine serogroups A, C, Y, W-135 (MENps-ASYW), measles virus, mumps virus, rubella virus vaccine (MMR), measles virus, mumps virus, rubella virus and varicella viurs vaccine (MMR-VAR), poliovirus attenuated live oral trivalent vaccine (OPV), pneumococcal (Streptococcus pneumoniae) vaccine (PNU), pneumococcal (Streptococcus pneumoniae) conjugate vaccine 7-valent (PNUcn-7), pneumococcal (Streptococcus pneumoniae) polysaccharide 23-valent vaccine (PNUps-23), poliovirus vaccine (POL), rabies vaccine (RAB), rabies vaccine human diploid cell culture (RAB-HDCV), rabies vaccine purified chick embryo cell culture (RAB-PCEC), respiratory syncytial virus vaccine (RSV), smallpox vaccine (SMA), smallpox (vaccinia virus) vaccine (SMAvac), tetanus toxoid and diphtheria toxoid (reduced antigen quantity for adults) vaccine (Td), toxoplasmosis (toxoplasm gondii) vaccine (TOX), typhoid (Salmonella typhi) vaccine (TPD), typhoid (Salmonella typhi) vaccine attenuated live oral Ty21a strain (TPDa), typhoid (Salmonella typhi) vaccine heat and phenol inactivated dried (TPD-HP), typhoid (Salmonella typhi) vaccine Vi capsular polysaccharide (TPD-Vi), Tuberculosis (Mycobacterium tuberculosis) vaccine, not BCG (TUB), varicella (chickenpox, varicella zoaster virus) vaccine (VAR).

Suitable parenteral compositions include, for example, adenosine, alprostadil, amikacin sulfate, azithromycin, bleomycin, ceftriaxone, ciprofloxacin, cisplatin, dacarbazine, daunorubicin HCl, deferoxamine mesylate, desmopressin acetate, diltiazem, dipyridamole, doxorubicin, enalaprilat, epirubicin, epoprostenol sodium, fluconazole, fludarabine phosphate, fludarabine phosphate, flumazenil, granisetron HCl, idarubicin HCl, ifosfamide, irinotecan HCl, leucovorin calcium, leuprolide acetate, levocarnitine, medroxyprogesterone acetate, mesna, methylprednisolone acetate, metoclopramide, mitoxantrone, nalbuphine HCl, norepinephrine bitartrate, octreotide acetate, ondansetron, oxaliplatin, oxytocin, paclitaxel, pamidronate disodium, pancuronium bromide, phenylephrine bromide, phenylephrine HCl, promethazine HCl, propofol, rocuronium bromide, sulfamethoxazole, sumatriptan succinate, tebutaline sulfate, testosterone cypionate, tobramycin, vecuronium bromide, vincristine sulfate, vinorelbine tartrate, and streptozocin sterile powder.

Suitable oral compositions include, for example, acetaminophen and codeine phosphate tablets, acetaminophen acetazolamide tablets, acyclovir capsules, amiloride HCl, amiodarone HCl, amlodipine besylate and benazepril capsules, amlodipine besylate tablets, amoxicillin and clavulanate potassium, amoxicillin, anagrelide, desogestrel and ethinyl estradiol tablets, asprin, atenolol, levonorgestrel and ethinyl estradiol tablets, azithromycin, baclofen, benazepril HCl, benzonatate, benztropine mesylate, bethamethasone valerate, bethamethasone dipropionate, bethanechol chloride, bicalutamide, bisoprolol fumarate, buproprion HCl, budesonide inhalation suspension, bumetanide, bupropion HCl, cabergoline, calcarb 600, calcitriol, calcium citrate tablets, norethindrone tablets, captopril and hydrochlorothiazide tablets, captopril, carbamazepine, carvedilol, cefaclor, cefadroxil, cefdinir, cefprozil, cephalexin, certagen, certirizine HCl, chlordiazepoxide HCl, chlorpheniramine maleate, chlorzoxazone, cholinoid, cilostazol, cimetidine HCl, cimetine, ciprofloxacin, citalopram, isotretinoin, clarithromycin, clemastine fumarate, clindamycin, clomiphene citrate, clomipramine HCl, clonazepam, clotrimazole, clozapine, cromolyn sodium, cyclobenzaprine HCl, cyclosporine, cyproheptadine HCl, danazol, demeclocycline HCl, desmopressin acetate, dexmethylphenidate HCl, dextroamphetamine sulfate, diazepam, diclofenac potassium, dicloxacillin, didanosine, ditiazem HCl, diphenhydramine HCl, dipyridamole, disopyramide phosphate, divalproex sodium, dorzolamide HCl, doxazosin mesylate, enalapril maleate, carbamazepine, estazolam, estradiol, estropipate, ethambutol HCl, ethosuximide, etodolac, famciclovir, famotidine, ferrous sulf, ferrous sulfate, fexofenadine HCl, finasteride, flecainide acetate, fluconazole, fludrocortisones acetate, fluocinonide, fluoxetine, flurbiprofen, flutamide, fluvoxamine, fosinopril sodium, furosemide, gabapentin, galantamine hydrobromide, gemfibrozil, glimepiride, glipizide, glucosamine sulfate, glyburide, haloperidol, hydralazine HCl, hydrochlorothiazide, hydrocondone bitartrate, hydroxychloroquine sulfate, hydroxyurea, hydroxyzine HCl, hydroxyzine pamoate, indomethacin, isoniazid, ketoconazole, ketoprofen, ketorolac tromethamine, labetalol HCl, lamotrigine, lansoprazole, leflunomide, leucovorin calcium, levetiracetam, lidocaine HCl, lisinopril, loperamide HCl, lorazepam, losartan potassiumlovastatin, mebendazole, medroxyprogesterone acetate, megestrol acetate, meloxicam, meperidine HCl, mercaptopurine, mesalamine, metformin HCl, methotrexate, methyldopa, methylprednisolone, metoclopramide, metoprolol tartrate, metronidazole, metronidazole, mexiletine, minocycline HCl, mirtazapine, misoprostol, moeipril HCl, mupirocin, mycophenolate mofetil, nabumetone, nadolol, naltrexone HCl, naproxen, nefazone HCl, neomycin sulfate, niacin, nifedipine, nimodipine, nizatidine, norethindrone acetate, nortriptyline HCl, nystatin, ofloxacin, omeprazole, ondansetron HCl, oxaprozin, oxazepam, oxybutynin chloride, oxycodone, oxycodone HCl, pantoprazole sodium, paroxetine, penicillin V potassium, pentoxifylline, phenylgesic, piroxicam, pramipexole dihydrochloride, pravastatin sodium, prazosin HCl, prednisolone, prochlorperazine maleate, propafenone HCl, propoxyphene HCl, propranolol HCl, protriptyline HCl, quinapril, quinidine sulfate, ramipril, ranitidine HCl, ribavirin, risperidone, ropinirole HCl, senna-S, sennagen, silver nitrate, simvastatin, sotalol HCl, sucralfate, tamoxifen citrate, tamsulosin HCl, terzosin HCl, terbinafine HCl, tetracycline HCl, theophylline, ticlopidine HCl, tolmetin sodium, topiramate, torsemide, tramadol HCl, trandolapril, trazodone HCl, tretinoin, ursodiol, valproic acid, venlafaxine HCl, verapamil HCl, warfarin sodium, zaleplon, and zolpidem tartrate.

Suitable nasal compositions include, for example, nasal sprays, antimigraine drugs, peptide drugs (hormone treatment), anaesthetics, antiemetics, sedatives, azelastin hydrochloride, oxymetazoline hydrochloride, pheynylephrine hydrochloride, saline solution, mometasone furoate, budesonide, ipratropium bromide, and cromolyn sodium nasal spray.

Suitable ocular compositions include, for example, lidocaine, proparacaine, tetracaine, ketorolac tromethamine ophthalmic solution, ketorolac tromethamine, naphazoline ophthalmic, brimonidine, azithromycin, bepotastine besilate, besifloxacin, betaxon, cosopt, diflupredate, lotemax, ranibizumab, bimatoprost, pegaptanib, ofloxacin, desamethasone, levofloxacin, unoprostone isopropyl ophthalmic solution, cyclosporine ophthalmic emulsion, salagen, travoprost ophthalmic solution, valganciclovir HCl, viroptic, cidofovir, verteporfin, vitrasert, vitravene, and ketotifen fumarate ophthalmic solution.

The method described herein can be performed using any suitable equipment or apparatus, and can be manual or automated. In one preferred aspect, the method is performed using the commercially available MILLIFLEX rapid microbiology detection system, which comprises a sample prep station, auto spray station, detection tower, image analyzer, CCD camera and computer with software (Millipore Corporation, Billerica, Massachusetts).

In another aspect, the invention is a sterile pharmaceutical composition (e.g., a vaccine, an ocular composition, a nasal composition, an oral composition, a parenteral composition) that has been screened for viable microorganisms using the methods described herein.

### EXEMPLIFICATION

### Creation of cryo- collection of ATCC strains and environmental strains from production site (surface or production personnel contact plates, contamination from bioburden and sterility tests)

Coming from either lyophilized culture (ATCC strains) or directly from plate (environmental isolates) the microorganisms were cultivated in either liquid Tryptic Soy Broth or on solid media (for example on Sabouraud Dextrose Agar) over an appropriate time period. Genotypic identification of each strain was performed using the MicroSeq System, Applied Biosystems. The culture was then centrifuged at 800 x G for 20-30 minutes and the pellet was resuspended in protective medium (Oxoid-CM67 containing 15% glycerine). The culture was diluted, CFU (colony forming units) were checked on solid media and filled into 2ml Nunc CryotubeTM Vials, storage at -80°C. Table 1 shows the complete list of microorganisms used.

**Table 1**

| Yeasts/Molds | Grampositive sporulating bacteria | Gramnegative bacteria | Grampositive cocci | Grampositive rods |
|---|---|---|---|---|
| *Aspergillus niger ATCC 16404* | *Bacillus subtilis* ATCC 6633 | *Escherichia coli* ATCC 8739 | *Staphylococcu s aureus* ATCC 6538 | *Propionibacteriu m acnes* HK-WST |
| *Candida albicans* ATCC 10231 | *B. licheniformis* HK-WST (2006) | *P. aeruginosa* ATCC 9027 | *Kocuria spez.* HK-WST | *C*. *afermentans* HK-WST |
| *Penicillium spez.* HK-WST | *Clostridium sporogenes* ATCC 11437 | *Acinetobacter lwoffii* HK-WST (2005) | *Staphylococcu s epidermidis* HK-WST | |
| | *Bacillus clausii* HK-WST | *Moraxella osloensis* HK-WST | *Staphylococcu s warneri* HK-WST | |
| | *Bacillus pumilus* HK-WST | | *S. capitis* HK-WST | |
| | *Bacillussphaericus* HK-WST | | *Micrococcusluteus* HK-WST | |
| | *Bacillus idriensis* HK-WST | | | |

### A. Stress factor study

Stress was evoked by the application of either UV-light (240-250µW/cm²), heat (50-70°C in a water bath) or by incubating the microorganisms in a dilution series of a parenteral drug product for 1-10 minutes each, taking aliquots every minute. Stress was directly applied to a fluid suspension of the tested microorganisms in a range under 100 CFU. Effects of stress were monitored by decrease in CFU, determined with plate count method and OD-measurements.

Plate count: The stressed microorganisms were plated on Tryptic Soy Agar monitoring the different effects of stress (stress applied for 1-10 minutes, taking aliquots every minute) by plating out aliquots of each minute. Results were counted out after 2-7 days (depending on strain, for example *E. coli* and *A. niger* were counted out after two days the latest, *P. acnes* could not be counted out earlier than after 6 days of incubation). The exact time necessary to lead to a decrease of the initially inoculated amount of CFU of more than 50% was measured.

OD-measurement (λ=600 nm): Overnight- cultures of the tested microorganisms were first stressed (the stress parameters which were determined in the ≥ 50% reduction study were applied), Tryptic Soy Broth or Fluid Thioglycollate Medium was inoculated (approximately 3-4 x 10⁶ CFU/ ml) and analyzed over a time frame of up to 8 hours (taking aliquots to measure optical density each hour). Incubation took place on a shaking table (only the aerobic strains). The stressed cultures were compared to the unstressed cultures.

### Results Stress factor study 50% reduction

For every microorganism out of the 22 strains, 3 stress-parameters were tested. The exact parameter (between 1 and 10 minutes) of each stress factor, which was necessary to lead to a decrease of the initially inoculated amount of CFU of more than 50% was measured. The three stress parameters tested were UV-light, heat and incubation of the microorganisms in a parenteral drug product for parenteral application. Heat, for example, causes damage on cytoplasmic membranes, RNA is denatured and this leads to the death of some cells. UV-irradiation causes mutations and a halt of DNA replication (M. Strus, Rocz Panstw Zakl Hig., 48 (3): 263-268 (1997)).

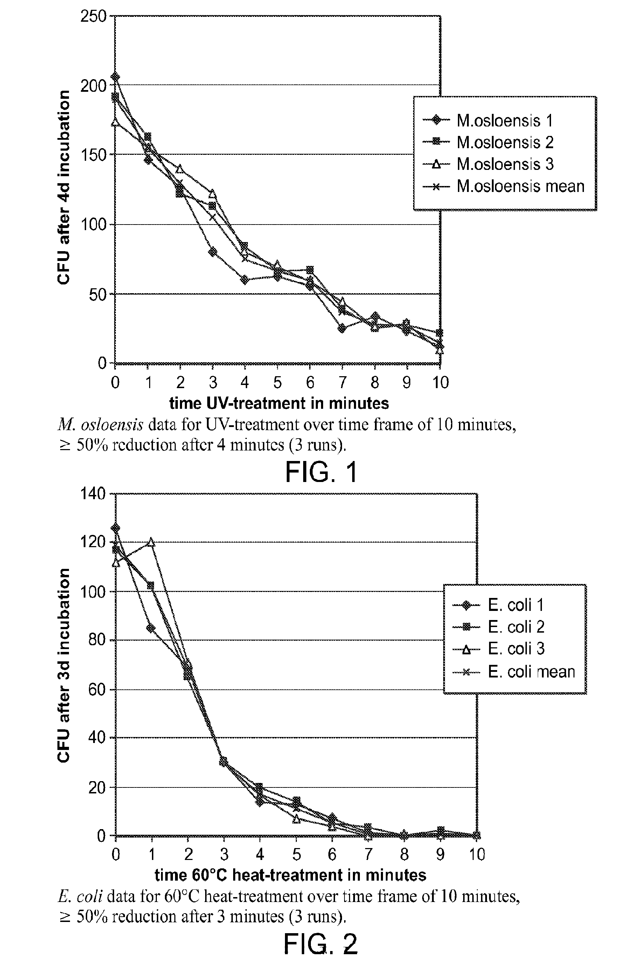

The application of a parenteral drug product leads to a chemical stress in the microbial cells- the antimicrobial properties of a parenteral drug product have been known for a long time. FIGS. 1-3 give examples of the data obtained for *Moraxella osloensis, Escherichia coli* and *Acinetobacter Iwoffii.* For each of the 22 strains of microorganisms the stress parameter necessary to reduce the initial inoculum by ≥50% was found.

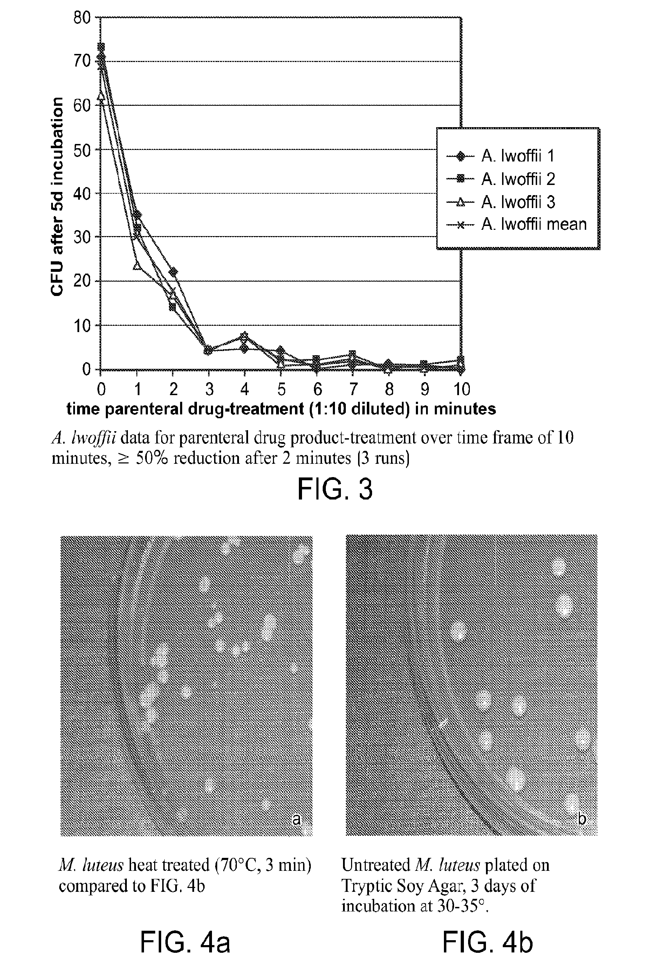

Observations of changed colony morphology (colonies grew slower, but regained normal colony size later on) were made in some cases. For example *Micrococcus luteus* showed a decrease in colony size [FIGS. 4a and 4b].

For culture media evaluation of the rapid sterility test it was important not only to use a broad range of microorganisms, but also to use these microorganisms in a stressed state. Some stress factors, such as chemical stress caused by a parenteral drug product and radiation stress caused by the application of UV-light decimated the amount of inoculated microorganisms. In contrast, a heat treatment led to an initially reduced growth rate in some of the tested microorganisms. Heat-injured cells have already been reported to take a time to recover from 3 to 4 hours-this was confirmed in this study (M. Warseck, Appl. Microbiol., 26: 919-922 (1973)). Heat stress is not useful for the sporulating bacteria, also other stress parameters are not feasible as sporulating bacteria show wide resistance towards stress (P. Setlow, J. Appl. Microbiol., 101 (3): 514-525 Review (2006)). For example *B. pumilus* is used as a radiation indicator bacterium (J. Wong, PDA J Pharm Sci Technol., 58(1):6-14 (2004)). In the case of the sporulating bacteria a different "stress" factor was used -these were stressed by nutrient depletion and therefore a higher spore content was induced.

The subsequent nutrient media evaluation and statistical analysis revealed that Schaedler Blood Agar was the best solid media for use in a sterility test. The t-test to analyze the differences between aerobic incubation at 20-25°C and at 30-35°C showed that there is a significant difference between the temperatures. As the difference was significant in 11 out of 40 cases it is necessary to validate both incubation temperatures in the rapid sterility test.

### OD-measurement

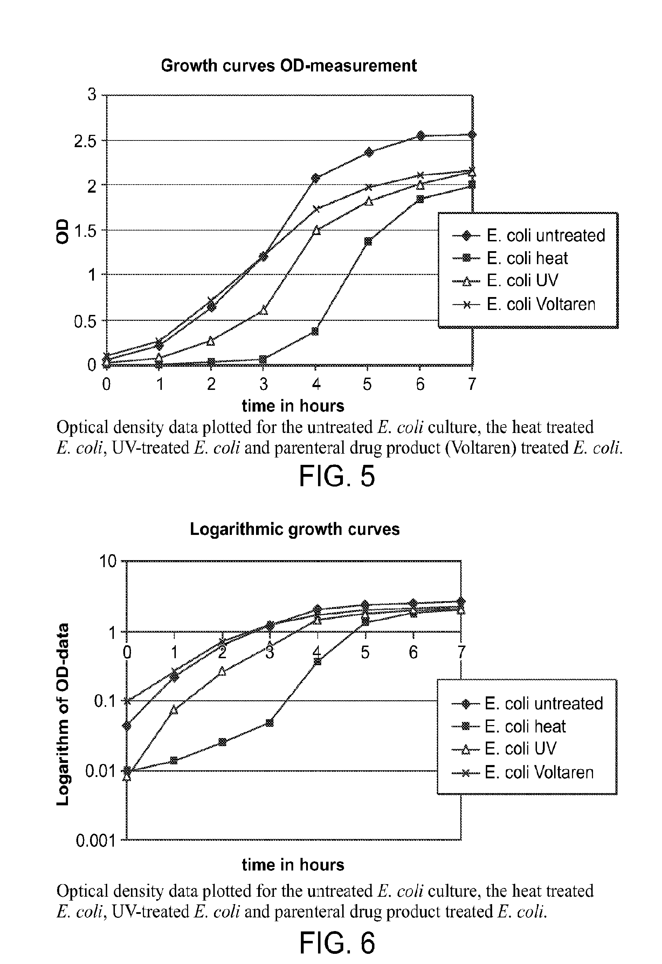

The stress parameters which were determined in the ≥50% reduction study were applied on each strain tested. The stressed inocula were always compared to the unstressed inoculum. The microorganisms (approximately 3-4 x 10⁶ CFU/ml as initial inoculum) were incubated either in Tryptic Soy Broth or Fluid Thioglycollate Medium and were incubated on a shaking table at 30-35°C (only the aerobic strains). Aliquots were taken each hour and the optical density (λ=600nm) was measured. Obtained data for the differently treated inocula (untreated, heat treated, treated with UV-light diluted in a parenteral drug product, time/parameter used from 50% reduction experiments) showed a slightly different growth curve in the normal plot of the OD-measured data [FIG.5].

Plotting the data logarithmically [FIG.6] shows an influence of heat-treatment on the growth curves of *E. coli* and makes the data independent of the amount of inoculum. As this is not easy to control precisely, the logarithmic plot has this as an advantage. There is no real difference in growth observed when the bacterial culture is stressed by either UV-light or by parenteral drug product-treatment (with the parameters determined in 50% reduction experiments).

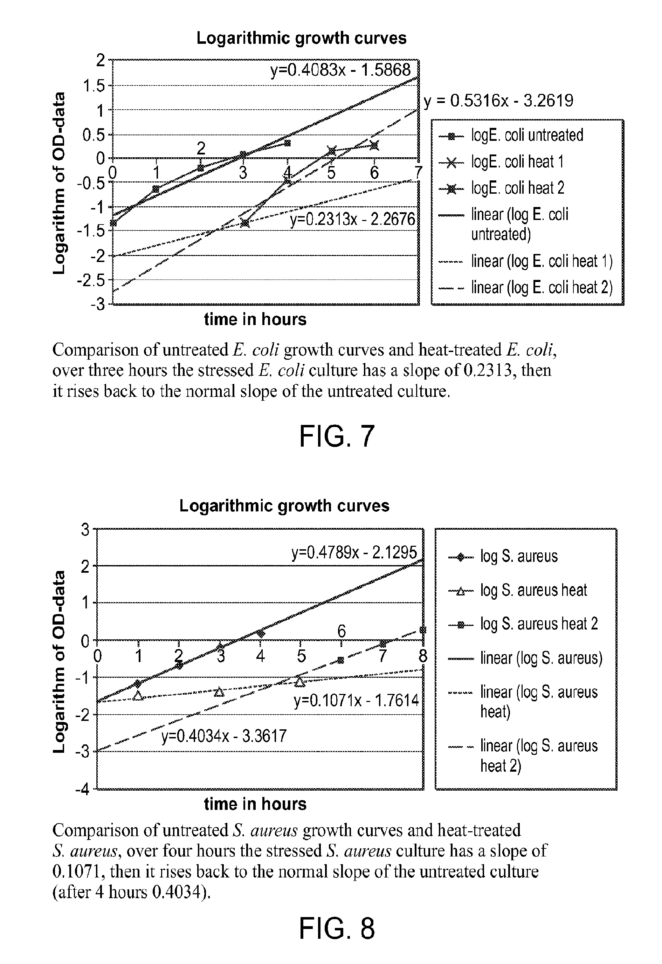

The comparison of the slopes show that only heat treatment (parameters as determined in 50% reduction experiments) had an influence on the growth rate of microorganisms and the microorganisms showed real stress. UV-light and dilution in a parenteral drug product (both "kill factors" and not "stress factors") were not used during culture media evaluation. The stress on the growth rate is in detail best shown by applying a straight line and by comparing the slopes. Reduced growth slopes were shown for all microbial strains in the chosen range. Examples illustrated herein are *E. coli, S. aureus, C. albicans* and *B. pumilus.* In the case of *E. coli,* the growth slope is reduced for approximately three hours, meaning this microorganism needs around three hours to resuscitate [FIG.7].

### S. aureus shows a reduced growth slope over the time frame of 4 hours [FIG.8]

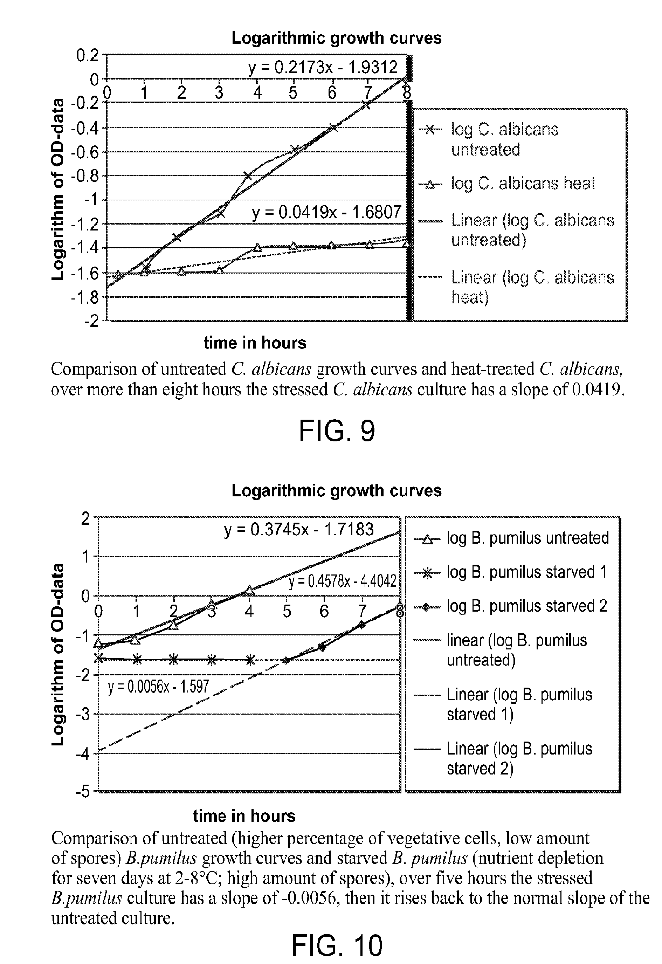

The yeast C. *albicans* showed an even longer enduring stress. The growth slope stayed reduced for more than eight hours, overnight the stressed culture regained the normal growth slope [FIG.9].

For grampositive sporulating bacteria heat stress is not feasible. Since the other stress factors UV-light and dilution in a parenteral drug product didn't show stress effects on the micro- organisms, another stress for the sporulating bacteria had to be found. For the *bacilli* and *clostrdia* a higher spore content in the bacterial suspension was evoked and the OD- measurement performed [FIG.10]. This sporulation was achieved by nutrient depletion and storing an overgrown microbial culture at 2-8°C for more than 6 days.

All microorganisms were tested in the described manner. Difficulties were observed in only three cases. The molds *Penicillium* and *Aspergillus* grow as mycelium in liquid medium, therefore no exact OD measurement was possible. As data are available for *C*. *albicans* and for all tested microorganisms, one can only assume that *Penicillium* and *Aspergillus* behave in the same manner. Therefore the tested parameters are taken for the culture media evaluation. In the other case, for *Propionibacterium acnes* (this strain grows better in FTM) the parameters for a ≥50% reduction, which were determined by plate count, could not be reproduced in the OD-measurement experiment. A time lag in growth was only observed when stress was omitted for a prolonged time frame as compared to the parameters from the ≥50% reduction experiment. *P. acnes* possibly showed different results in the OD-measurement due to higher oxygen stress. For OD-measurement, aliquots were taken every hour, leading to a certain oxygen stress for *P. acnes.* The resulting data of the stress factor study are summarized in Table 2.

**Table 2: List of microorganisms including their defined stress parameters**

| **Yeasts/Molds :** | **Grampositive sporulating bacteria:** | **Gramnegative bacteria:** | **Grampositive cocci:** | **Grampositive rods:** |
|---|---|---|---|---|
| *Aspergillus niger* ATCC 16404 no OD measurement possible; 60°C, 3 min | *Bacillus subtilis* ATCC 6633 starvation (6d) | *Escherichia coli* ATCC 8739 60°C, 3 min | *Staphylococcus aureaus* ATCC 6538 60°C, 4 min | *Propionibacterium acnes* HK-WST 60°C, 1 min; not reproducible in OD measurement |
| *Candida albicans* ATCC 10231 60°C, 2 min | *B. licheniformis* HK-WST (2006) starvation (6d) | *P. aeruginosa* ATCC 9027 60°C, 2 min | *Kocuriaspez.* HK-WST 60°C, 2 min | *C. afermentans* HK-WST 60°C, 2 min |
| *Penicillium spez.* HK-WST no OD measurement possible; 50°C, 3 min | *Clostridiumsporogenes* ATCC 11437 starvation (15d) | *Acinetobacterlwoffi* HK-WST (2005) 60°C, 2 min | *Staphyloccus epidermidis* HK-WST 60°C, 2 min | |
| | *Bacillus clausii* HK-WST (7d) | *Moraxella osloensis* HK-WST 60°C, 3 min | *Staphylococcus warneri* HK-WST 60°C, 3 min | |
| | *Bacillus pumilus* HK-WST starvation (7d) | | *S. capitis* HK-WST 60°C, 4 min | |
| | *Bacillus sphaericus* HK-WST starvation (21d) | | *Micrococcus luteus* HK-WST 70°C, 3 min | |
| | *Bacillus idriensis* HK-WST starvation (7d) | | | |

The stress parameters heat and nutrient depletion were, therefore, used in the culture media evaluation. The chosen stress factors resemble the stress possibly present in the production process of sterile drug products.

### B. Growth Promotion Study

Culture media to be tested were inoculated with the microorganism (stressed and unstressed state) with an approximate amount of 10-100 CFU. The experiment was conducted using 5 replicates for each incubation parameter (incubation parameters are: 20-25°C and 30-35°C aerobic incubation 30-35°C anaerobic incubation). Results were visually counted after 2-7 days of incubation. Resulting raw data were grouped in 6 groups for each microorganism:
1. 20-25°C aerobic incubation - stressed microorganism,
2. 20-25°C aerobic incubation - unstressed microorganism,
3. 30-35°C aerobic incubation - stressed microorganism,
4. 30-35°C aerobic incubation - unstressed microorganism,
5. 30-35°C anaerobic incubation - stressed microorganism,
6. 30-35°C anaerobic incubation - unstressed microorganism.

The tested nutrient media are grouped together in subgroups.

### List of solid nutrient media for preselection (growth promotion test with 10 strains, unstressed

- FTM-A (Fluid Thioglycollate Medium containing additional 10g/L Agar, leading to an end concentration of 1.075% Agar), Amimed, Allschwil, Switzerland
- BHI (Brain Heart Infusion Agar), γ-irradiated, heipha, Eppelheim, Germany
- Difco Brewer Anaerobic Agar, γ-irradiated, heipha, Eppelheim, Germany
- R2A Agar, Oxoid, Great Britain
- Schaedler Blood Agar, γ-irradiated heipha, Eppelheim, Germany
- Caso-Agar ICR (Tryptic Soy Agar), γ-irradiated, heipha, Eppelheim, Germany
- Columbia Agar 5% Blood, BioMerieux, France
- CDC Anaerobic Blood Agar, γ-irradiated, heipha, Eppelheim, Germany

### List of solid nutrient media for final study (growth promotion test with 22 strains, unstressed and stressed state)

- Difco Brewer Anaerobic Agar, γ-irradiated, heipha, Eppelheim" Germany
- Schaedler Blood Agar, γ-irradiated, heipha, Eppelheim, Germany
- Caso-Agar ICR (Tryptic Soy Agar), γ-irradiated heipha, Eppelheim, Germany
- CDC Anaerobic Blood Agar, γ-irradiated, heipha, Eppelheim, Germany
All γ-irradiated media were supplemented accordingly to sustain the irradiation process.

### Nutrient media evaluation

The nutrient media evaluation study was done in two parts: the first preselection (growth promotion test with 10 strains, unstressed, tests on the eight agars) led to a reduction down to four media, which meant that only these media came into closer consideration. FTM-A Agar, Brain Heart Infusion Agar, R2A Agar and Columbia Agar 5% Blood were excluded in this preselection.

In the nutrient media evaluation the following four media were tested in detail: Tryptic Soy Agar, CDC Anaerobic Blood Agar, Schaedler Blood Agar and Difco Brewer Anaerobic Agar. The resulting data, for each of the 22 strains which were inoculated both in a stressed and in an unstressed state, were grouped and statistically analyzed using an ANOVA. The three incubation parameters 20-25°C and 30-35°C aerobic incubation and 30-35°C anaerobic incubation and the two different stress states for each microorganism (stressed and unstressed) lead to the formation of 6 groups for each microorganism (multiplied by 22 strains). An ANOVA of each of these groups was performed, credits for good growth promoting properties were summarized for the 22 microorganisms for each agar. One credit was given to the group/agar, if it achieved the highest count. Groups/agar without significant difference to this highest count agar also gained one credit.

In the 20-25°C aerobic incubation-group Schaedler Blood Agar gathered 30 credits, the CDC Anaerobic Blood Agar 27 credits, Tryptic Soy Agar gathered 24 and Difco Brewer Anaerobic Agar 17 [Table 3A and 3B]. *P. acnes* and *Cl. sporogenes* gathered no credits, as they don't grow aerobically. Stressed *A. niger, B. pumilis, B. sphaerieus* and *B. idriensis* got 0 credits due to low counts on all tested agars (0-5 CFU).

**Table 3A: Total count of credits for the unstressed microorganisms cultivated at 20-25°C aerobically.**

| | **TSA** | **CDC** | **Schaedler** | **Brewer** |
|---|---|---|---|---|
| *A. niger* | 1 | 1 | 1 | 1 |
| *C. albicans* | 1 | 1 | 1 | 1 |
| *B. subtilis* | 1 | 1 | 1 | 1 |
| *E. coli* | 1 | 0 | 1 | 1 |
| *S. aureus* | 1 | 1 | 1 | 1 |
| *P. aeruginosa* | 0 | 1 | 1 | 1 |
| *A.Iwoffii* | 1 | 1 | 1 | 0 |
| *B. liceniformis* | 1 | 1 | 0 | 0 |
| *M. luteus* | 0 | 1 | 1 | 0 |
| *Cl. sporogenes* | 0 | 0 | 0 | 0 |
| *P. acnes* | 0 | 0 | 0 | 0 |
| *B. clausii* | 1 | 1 | 1 | 0 |
| *C. afermentans* | 1 | 1 | 1 | 0 |
| *S. epidermidis* | 1 | 1 | 1 | 1 |
| *Kocuria spez.* | 0 | 0 | 0 | 1 |
| *Penicillium spez.* | 0 | 1 | 1 | 0 |
| *S. warneri* | 0 | 1 | 1 | 0 |
| *B. pumilus* | 1 | 1 | 1 | 1 |
| *S. capitis* | 0 | 0 | 1 | 0 |
| *B. sphaericus* | 1 | 0 | 1 | 0 |
| *B. idriensis* | 1 | 1 | 1 | 0 |
| *M. osloensis* | 1 | 1 | 0 | 0 |
| *total* | 14 | 15 | 17 | 9 |

**Table 3B: Total count of credits for the stressed microorganisms cultivated at 20-25°C aerobically.**

| | **TSA** | **CDC** | **Schaedler** | **Brewer** |
|---|---|---|---|---|
| *A. niger* | 0 | 0 | 0 | 0 |
| *C. albicans* | 0 | 0 | 1 | 0 |
| *B. subtilis* | 1 | 1 | 1 | 1 |
| *E. coli* | 0 | 1 | 1 | 1 |
| *S. aureus* | 1 | 1 | 1 | 0 |
| *P. aeruginosa* | 0 | 1 | 1 | 1 |
| *A. Iwoffii* | 1 | 0 | 0 | 1 |
| *B. liceniformis* | 1 | 1 | 1 | 1 |
| *M. luteus* | 1 | 1 | 1 | 0 |
| *Cl. sporogenes* | 0 | 0 | 0 | 0 |
| *P. acnes* | 0 | 0 | 0 | 0 |
| *B. clausii* | 1 | 1 | 1 | 0 |
| *C. afermentans* | 1 | 0 | 0 | 0 |
| *S. epidermidis* | 0 | 0 | 1 | 0 |
| *Kocuria spez.* | 1 | 1 | 1 | 1 |
| *Penicillium spez.* | 1 | 1 | 1 | 1 |
| *S. warneri* | 0 | 1 | 1 | 0 |
| *B. pumilus* | 0 | 0 | 0 | 0 |
| *S. capitis* | 0 | 1 | 1 | 0 |
| *B. sphaericus* | 0 | 0 | 0 | 0 |
| *B. idriensis* | 0 | 0 | 0 | 0 |
| *M. osloensis* | 1 | 1 | 0 | 1 |
| *total* | 10 | 12 | 13 | 8 |

In the 30-35°C aerobic incubation-group Schaedler Blood Agar gathered 28 credits, Tryptic Soy Agar gathered 28 credits, CDC Anaerobic Blood Agar 27 credits and Difco Brewer Anaerobic Agar 24 [Table 4A and 4B]. *P. acnes* and *Cl. sporogenes* gathered no credits, as they don't grow aerobically. Stressed *B. idriensis* and *M.osloensis* got 0 credits due to low counts (0-5 CFU).

**Table 4A: Total count of credits for the stressed microorganisms cultivated at 30-35°C**

| | **TSA** | **CDC** | **Schaedler** | **Brewer** |
|---|---|---|---|---|
| *A. niger* | 1 | 1 | 1 | 1 |
| *C. albicans* | 1 | 1 | 1 | 1 |
| *B. subtilis* | 1 | 1 | 1 | 1 |
| *E. coli* | 1 | 0 | 0 | 1 |
| *S. aureus* | 1 | 1 | 1 | 1 |
| *P. aeruginosa* | 1 | 1 | 1 | 1 |
| *A. Iwoffii* | 1 | 0 | 1 | 0 |
| *B. liceniformis* | 1 | 1 | 1 | 1 |
| *M. luteus* | 0 | 1 | 1 | 1 |
| *Cl. sporogenes* | 0 | 0 | 0 | 0 |
| *P. acnes* | 0 | 0 | 0 | 0 |
| *B. clausii* | 1 | 0 | 0 | 0 |
| *C. afermentans* | 1 | 1 | 1 | 1 |
| *S. epidermidis* | 1 | 1 | 1 | 1 |
| *Kocuria spez.* | 1 | 1 | 1 | 1 |
| *Penicillium spez.* | 0 | 1 | 0 | 1 |
| *S. warneri* | 1 | 0 | 0 | 1 |
| *B. pumilus* | 0 | 1 | 1 | 1 |
| *S. capitis* | 0 | 0 | 0 | 1 |
| *B. sphaericus* | 0 | 0 | 1 | 0 |
| *B. idriensis* | 1 | 0 | 0 | 0 |
| *M. osloensis* | 1 | 1 | 0 | 0 |
| *total* | 15 | 13 | 13 | 15 |

**Table 4B: Total count of credits for the unstressed microorganisms cultivated at 30-35°C**

| | **TSA** | **CDC** | **Schaedler** | **Brewer** |
|---|---|---|---|---|
| *A. niger* | 1 | 1 | 1 | 1 |
| *C. albicans* | 0 | 0 | 1 | 1 |
| *B. subtilis* | 1 | 1 | 1 | 1 |
| *E. coli* | 1 | 1 | 1 | 1 |
| *S. aureus* | 1 | 1 | 1 | 0 |
| *P. aeruginosa* | 1 | 1 | 1 | 1 |
| *A. Iwoffii* | 1 | 1 | 0 | 1 |
| *B. liceniformis* | 0 | 0 | 0 | 0 |
| *M. luteus* | 0 | 1 | 1 | 0 |
| *Cl. sporogenes* | 0 | 0 | 0 | 0 |
| *P. acnes* | 0 | 0 | 0 | 0 |
| *B. clausii* | 1 | 0 | 1 | 0 |
| *C. afermentans* | 0 | 1 | 1 | 0 |
| *S. epidermidis* | 1 | 1 | 1 | 0 |
| *Kocuria spez.* | 1 | 1 | 1 | 1 |
| *Penicillium spez.* | 0 | 1 | 0 | 0 |
| *S. warneri* | 1 | 0 | 1 | 0 |
| *B. pumilus* | 1 | 1 | 1 | 1 |
| *S. capitis* | 0 | 1 | 1 | 0 |
| *B. sphaericus* | 1 | 1 | 1 | 1 |
| *B. idriensis* | 0 | 0 | 0 | 0 |
| *M. osloensis* | 1 | 0 | 0 | 0 |
| *total* | 13 | 14 | 15 | 9 |

In the 30-35°C anaerobic incubation-group CDC Anaerobic Blood Agar gathered 25 credits, the Schaedler Blood Agar 25 credits, Tryptic Soy Agar 21 and Difco Brewer Anaerobic Agar gathered 17 credits [Table 5A and 5B]. Stressed *B*. *idriensis* got 0 credits due to low count (0-5 CFU).

**Table 5A: Total count of credits for the unstressed microorganisms cultivated at 30-35°C anaerobically. Not all microorganisms grow anaerobically, therefore no count was given.**

| | **TSA** | **CDC** | **Schaedler** | **Brewer** |
|---|---|---|---|---|
| *A. niger* | 0 | 0 | 0 | 0 |
| *C. albicans* | 0 | 0 | 0 | 1 |
| *B. subtilis* | 1 | 1 | 1 | 1 |
| *E. coli* | 1 | 1 | 1 | 1 |
| *S. aureus* | 1 | 1 | 0 | 1 |
| *P. aeruginosa* | 1 | 1 | 1 | 1 |
| *A. Iwoffii* | 0 | 0 | 0 | 0 |
| *B. liceniformis* | 1 | 1 | 1 | 0 |
| *M. luteus* | 0 | 0 | 0 | 0 |
| *Cl. sporogenes* | 1 | 1 | 1 | 0 |
| *P. acnes* | 1 | 1 | 1 | 0 |
| *B. clausii* | 1 | 0 | 0 | 0 |
| *C. afermentans* | 0 | 0 | 0 | 0 |
| *S. epidermidis* | 1 | 1 | 1 | 1 |
| *Kocuria spez.* | 1 | 1 | 1 | 1 |
| *Penicillium spez.* | 0 | 0 | 0 | 0 |
| *S. warneri* | 1 | 1 | 1 | 1 |
| *B. pumilus* | 0 | 1 | 0 | 1 |
| *S. capitis* | 0 | 0 | 0 | 1 |
| *B. sphaericus* | 1 | 1 | 1 | 1 |
| *B. idriensis* | 1 | 1 | 1 | 0 |
| *M. osloensis* | 0 | 0 | 0 | 0 |
| *total* | 13 | 13 | 11 | 11 |

**Table 5B: Total count of credits for the stressed microorganisms cultivated at 30-35°C anaerobically. Not all microorganisms grow anaerobically, therefore no count was given.**

| | **TSA** | **CDC** | **Schaedler** | **Brewer** |
|---|---|---|---|---|
| *A. niger* | 0 | 0 | 0 | 0 |
| *C. albicans* | 0 | 0 | 1 | 0 |
| *B. subtilis* | 1 | 1 | 1 | 1 |
| *E. coli* | 1 | 1 | 1 | 1 |
| *S. aureus* | 1 | 1 | 1 | 1 |
| *P. aeruginosa* | 0 | 1 | 1 | 1 |
| *A. Iwoffii* | 0 | 0 | 0 | 0 |
| *B. liceniformis* | 1 | 1 | 1 | 1 |
| *M. luteus* | 0 | 0 | 0 | 0 |
| *Cl. sporogenes* | 0 | 1 | 1 | 0 |
| *P. acnes* | 1 | 1 | 1 | 0 |
| *B. clausii* | 1 | 0 | 1 | 0 |
| *C. afermentans* | 0 | 0 | 0 | 0 |
| *S. epidermidis* | 0 | 1 | 1 | 0 |
| *Kocuria spez.* | 0 | 0 | 0 | 0 |
| *Penicillium spez.* | 0 | 0 | 0 | 0 |
| *S. warneri* | 0 | 1 | 1 | 0 |
| *B. pumilus* | 1 | 1 | 1 | 1 |
| *S. capitis* | 0 | 1 | 1 | 0 |
| *B. sphaericus* | 1 | 1 | 1 | 0 |
| *B. idriensis* | 0 | 0 | 0 | 0 |
| *M. osloensis* | 0 | 0 | 0 | 0 |
| *total* | 8 | 12 | 14 | 6 |

The data show that Schaedler Blood Agar had the best growth promoting properties for the aerobic conditions, in the anaerobic condition it gained the same amount of credits as CDC Anaerobic Blood Agar. Therefore the Schaedler Blood Agar was selected as the suitable agar for the 22 tested strains and for the three tested incubation parameters. The composition of Schaedler Blood Agar (13) (modified by heipha, Eppelheim, Germany) is as follows: Caseine peptone 10g, soy flour peptone 1g, meat peptone 2g, meat exact 1g, yeast extract 5g, glucose 2g, NaCl 5g, dipotassium hydrogen phosphate 2.5g, agar bacteriological grade 14g, sheep blood 50ml, amino acids, buffer, hemin, vitamin K, gamma-irradiated: 9-20kGy.

### Differences between aerobic incubation at 20-25°C and at 30-35°C

A t-test was performed using the data from the nutrient media evaluation to show if there is a significant difference between the incubation temperatures 20-25°C and 30-35°C (aerobic incubation).

The t-test was done on 40 groups consisting of all aerobic microorganisms (without *Cl. sporugeloes* and *P. acnes*) in both stressed and unstressed state. In 11 groups out of the 40 groups a significant difference occurred. 5 times the incubation parameter 30-35°C showed higher amounts of colony forming units (coming from the same inoculm) compared to the 20-25°C group. The group 20-25°C produced higher values in six cases. The t-test showed that there is a significant difference between the temperatures. As the difference was significant in 11 out of 40 cases it is necessary to validate both incubation temperatures in the rapid sterility test. These results stress the importance of both incubation temperatures not only for the traditional sterility test but also for the rapid sterility test.

### Statistical Analysis of Data

For statistical analysis of the raw data the following methods implemented in Minitab® Release 14.20 Statistical Software was used.

The ANOVA (Analysis of variance) compares means. An ANOVA is similar to regression analysis, because it is used to investigate and model the relationship between a response variable and one or more independent variables (between groups). It uses hypothesis testing, this means a null hypothesis is tested. The ANOVA results in a p-value. The p-value stands for the probability of making a Type 1 error, or rejecting the null hypothesis when it is true. The cutoff value is 0.05 - the null hypothesis is rejected, when the p-value is below 0.05 corresponding to a confidence limit of 95%.

Prerequisites for using an ANOVA analysis are:
- normal distribution of the four subgroups (the nutrient media), p-value≥0.05
- test for equal variances using Bartlett's Test: p-value≥0.05 and
- Levene's Test: p-value≥0.05.

ANOVA'S p-value ≥ 0.05 means there is no significant difference between the subgroups / the agars, p-value <0.05 significant difference between subgroups / the agars. Every agar which showed no significant difference to the highest mean value (including the one having the highest mean) was rated with one credit. An agar which showed a significant difference to the highest mean value got no credit. Outlier analysis was performed using the Dixon's Test (W. J. Dixon, Ann. Math. Statist., 21:488-506 (1950); W. J. Dixon, Biometrics, 9:74-89 (1953); USP <1010> "Analytical data-interpretation and treatment," Pharmacopeial Forum. USP 30-NF through Second Supplement The United States Pharmacopeial Convention, Inc.). For this the N values comprising the set of observations under examination were arranged in ascending order: X₁ < X₂ < ... < X_{N}. Then the statistic experimental Q-value (Qexp) was calculated. This is a ratio defined as the difference of the suspect value from its nearest one divided by the range of the values (Q: rejection quotient). Thus, for testing x₁ or X_{N} (as possible outliers) the following Qexp values were used:

The obtained Qexp value was compared to a critical Q-value (Qcrit) found in tables. If Qexp > Qcrit, then the suspect value can be characterized as an outlier and it can be rejected. If not, the suspect value had to be retained and used in all subsequent calculations. Other methods to be able to perform the ANOVA were the following: Subgroups (the four nutrient media inside a group) which didn't follow normal distribution and were significantly lower than the other subgroups were excluded, because they were irrelevant to the ANOVA. If CFU-values of the subgroups inside a group were too low (0-5 CFU), the final result of 4 times 0 credits was given. In all other cases a retest had to be performed. A t-test (also using Minitab® Release 14.20 Statistical Software) is used to compare means of two samples; the t-test compares the actual difference between two means in relation to the variation in data (expressed as the standard deviation of the difference between the means).

### C. General description of the Millipore Milliflex® Rapid Microbiology Detection System

Samples under test (diluted in 100 ml of rinsing fluid to assure adequate distribution of micro- organisms on membrane) will be filtered over Milliflex@ Rapid filter using Mifliflex® PLUS pump, a possible contamination will be trapped on the filter membrane (pore size: 0,45µm).

Following filtration the filter will be applied to solid nutrient media using the Milliflex® system, in which the sterile Milliflex® filter clicks on the Milliflex® cassette and the funnel breaks off. Due to this Milliflex® system to transfer the membrane onto the nutrient medium, there will be low or no risk of secondary contamination through membrane handling. As the cassettes are closed tightly the risk for secondary contamination during incubation is low. By incubating the filters on the Milliflex® cassettes, growth of potential contaminant(s) to micro-colonies can take place. At the end of the incubation time, filters will be separated from the Milliflex® cassettes and applied onto the Autospray Station inside a laminar flow hood. The steps after incubation are not critical steps regarding secondary contamination, since micro-colonies have to have a certain size (approximately: 10-100 yeast cells, 1000 bacterial cells) which for detection in the Milliflex® Rapid. If an environmental or operator-derived microorganism is added to the membrane during the steps after incubation, it will not be detected due to the lack of incubation and therefore the lack of a certain amount of ATP which is necessary for detection.

The AutoSpray Station sprays the ATP releasing agent and afterwards the bioluminescence reagent onto the membrane.

The reaction chemistry shown here is the basis of Milliflex® Rapid detection (W.D. McElroy, Adv. Enzymol. Relat. Areas. Mol. Biol., 25:119-166 (1963)):
Luciferan + ATP Luciferase, Mg²⁺ →Oxyluciferin + AMP + hv (λ=562nm) (Mg2+ means Magnesium, ATP Adenosine Triphosphate, AMP Adenosine Monophosphate, hv emitted photon, λ wavelength)

Fast transfer of the sprayed membrane from the Autospray Station to the Milliflex® Rapid Detection Tower is necessary in order to catch the maximum of emitted photons. The possible light signals are detected with a CCD-camera (charge coupled device) and the algorithm of the Milliflex®, Rapid software calculates the amount of micro-colonies.

### No bioluminescent background in Milliflex® Rapid detection

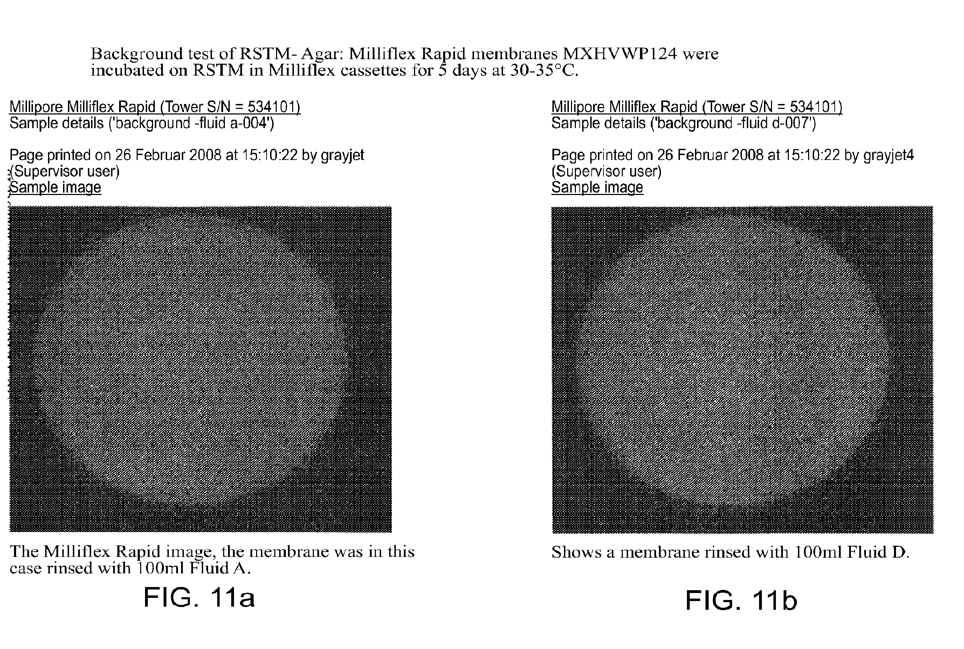

As the Schaedler Blood Agar will be used in the Milliflex® Rapid Microbiology Detection System, a test was performed to determine if this medium causes bioluminescent background. For this, the media cassettes were incubated with a MXHVWP124 membrane (Polyvinylidenefluoride membrane, pore size 0.45µm) which had been rinsed with either 100ml of Fluid A or Fluid D. The incubation temperature was 30-35°C, incubated for 5 days. Figure 11 shows that there is no bioluminescent background coming from the Schaedler Blood Agar, meaning no disturbing ATP is left in the gamma-irradiated nutrient medium. Schaedler Blood Agar is suitable for use in the Milliflex® Rapid System.

## Claims

1. A method for sterility testing of a liquid pharmaceutical composition comprising:
(a) providing a filterable pharmaceutical composition;
(b) filtering the pharmaceutical composition to provide at least three filter membranes upon which the pharmaceutical composition filtrand is deposited;
(c) placing the at least three filter membranes onto solid culture media to produce at least three filtrand cultures;
(d) culturing (i) at least one filtrand culture under aerobic conditions at 20-25°C; (ii) at least one filtrand culture under aerobic conditions at 30-35°C; and (iii) at least one of filtrand culture under anaerobic conditions at 30-35°C; with the proviso that none of the filtrand cultures are cultured for a period of more than about 13 days; and
(e) detecting a viable microorganism cell, micro-colony or colony on a membrane, wherein the presence of a viable microorganism cell, micro-colony or colony on the membrane indicates the presence of a viable microorganism in the pharmaceutical composition.

2. The method of claim 1, wherein (b) further comprises filtering a wash solution after the pharmaceutical composition is filtered.

3. The method of claim 1 or 2 wherein the membrane(s) is/are a polyvinylidene fluoride membrane, glass fibre membrane, polycarbonate membrane, polyethylene terephthalate membrane, mixed cellulose ester (cellulose acetate and cellulose nitrate), phosphocellulose membrane, DEAE membrane, nylon mesh membrane, or polytetrafluoroethylene membrane.

4. The method of any one of the preceding claims, wherein the membrane(s) has/have a pore size of about 0.45 µm.

5. The method of any one of the preceding claims, wherein the solid culture media is selected from the group consisting of FTM-A (fluid thioglycollate medium containing 1.075% agar (final concentration)), BHI (brain heart infusion agar), Difco brewer anaerobic agar, R2A agar, Schaedler blood agar, Caso-agar ICR (tryptic soy agar), Columbia agar 5% blood, and CDC anaerobic blood agar.

6. The method of any one of the preceding claims, wherein in (d) the filtrand cultures are cultured for a period of time sufficient for the production of a detectable amount of adenosine triphosphate.

7. The method of claim 6, wherein the filtrand cultures are cultured for a period of about 2 to about 7 days.

8. The method of claim 7, wherein the filtrand cultures are cultured for a period of 5 days.

9. The method of any one of the preceding claims, wherein in (e) a viable microorganism cell, micro-colony or colony is detected using a luminescence assay.

10. The method of claim 9, wherein the luminescence assay: (i) detects adenosine triphosphate produced by a viable microorganism cell, micro-colony or colony on the membrane; (ii) comprises a luciferase assay; or (iii) detects a nucleic acid hybridization product formed between a probe and a nucleic acid endogenous to a microorganism, for example comprising a peroxidase reaction.

11. The method of claim 9 or 10, wherein luminescence is detected using a charged coupled device camera and image analysis software.

12. The method of any one of the preceding claims, which is performed on a rapid microbiology detection system which comprises a sample prep station, an auto spray station, a detection tower, an image analyzer, a CCD camera and a computer with software.

13. The method of any one of the preceding claims, wherein the pharmaceutical composition is a parenteral composition, an oral composition, a nasal composition, or an ocular composition.

14. The method of claim 13, wherein the pharmaceutical composition is a vaccine, for example selected from the group consisting of anthrax vaccine; tuberculosis vaccine; Borreliosis vaccine; diphtheria toxoid and tetanus toxoid vaccine; diphtheria toxoid and tetanus toxoid and pertussis vaccine; diphtheria toxoid and tetanus toxoid and acellular pertussis vaccine; diphtheria toxoid and tetanus toxoid and acellular perussis and *Haemophilus influenzae* type b conjugate vaccine; diphtheria toxoid and tetanus toxoid and acellular perussis and *Haemophilus influenzae* type b conjugate and poliovirus inactivated vaccine; hepatitis A virus vaccine; hepatitis A virus and hepatitis B virus vaccine; hepatitis B virus vaccine; *Helicobacter pylori* vaccine; *Haemophilus influenzae* type b vaccine; influenza virus vaccine; poliovirus vaccine; meningococcal (*Neisseria meningitidis*) vaccine; measles virus, mumps virus, rubella virus vaccine; measles virus, mumps virus, rubella virus and varicella virus vaccine; pneumococcal (*Streptococcus pneumoniae*) vaccine; rabies vaccine; respiratory syncytial virus vaccine; smallpox vaccine; toxoplasmosis (*Toxoplasma gondii*) vaccine; typhoid (*Salmonella typhi*) vaccine; tuberculosis (*Mycobacterium tuberculosis*) vaccine; and varicella (chickenpox, Varicella zoster virus) vaccine; and preferably wherein said vaccine is avian influenza vaccine or swine influenza vaccine.

15. A method for sterility testing of a liquid pharmaceutical composition comprising:
(a) providing a filterable pharmaceutical composition;
(b) filtering the pharmaceutical composition to provide at least three membranes upon which the pharmaceutical composition filtrand is deposited;
(c) placing the at least three filters/membranes onto solid culture media to produce at least three filtrand cultures;
(d) culturing (i) at least one filtrand culture under aerobic conditions at 20-25°C; (ii) at least one filtrand culture under aerobic conditions at 30-35°C; and (iii) at least one of filtrand culture under anaerobic conditions at 30-35°C; with the proviso that none of the filtrand cultures are cultured for a period of more than about 13 days; and
(e) detecting adenosine triphosphate (ATP) on the membrane, wherein the presence of ATP on a membrane indicates the presence of a viable microorganism in the pharmaceutical composition.

## Patentansprüche

1. Verfahren zum Testen einer flüssigen pharmazeutischen Zusammensetzung auf Sterilität, bei dem man:
(a) eine filtrierbare pharmazeutische Zusammensetzung bereitstellt;
(b) die pharmazeutische Zusammensetzung filtriert, unter Bereitstellung von mindestens drei Filtermembranen, auf denen sich der Filtrierrückstand der pharmazeutischen Zusammensetzung abscheidet;
(c) die mindestens drei Filtermembranen auf feste Kulturmedien gibt und so mindestens drei Filtrierrückstandkulturen herstellt;
(d) (i) mindestens eine Filtrierrückstandkultur unter aeroben Bedingungen bei 20-25°C kultiviert; (ii) mindestens eine Filtrierrückstandkultur unter aeroben Bedingungen bei 30-35°C kultiviert und (iii) mindestens eine Filtrierrückstandkultur unter anaeroben Bedingungen bei 30-35°C kultiviert; mit der Maßgabe, dass keine der Filtrierrückstandkulturen länger als etwa 13 Tage lang kultiviert wird; und
(e) eine lebensfähige Mikroorganismuszelle, Mikrokolonie oder Kolonie auf einer Membran nachweist, wobei das Vorhandensein einer lebensfähigen Mikroorganismuszelle, Mikrokolonie oder Kolonie auf der Membran das Vorhandensein eines lebensfähigen Mikroorganismus in der pharmazeutischen Zusammensetzung anzeigt.

2. Verfahren nach Anspruch 1, wobei (b) weiterhin das Filtrieren einer Waschlösung nach dem Filtrieren der pharmazeutischen Zusammensetzung umfasst.

3. Verfahren nach Anspruch 1 oder 2, wobei es sich bei der Membran/den Membranen um eine Polyvinylidenfluoridmembran, eine Glasfasermembran, eine Polycarbonatmembran, eine Polyethylenterephthalatmembran, einen gemischten Celluloseester (Celluloseacetat und Cellulosenitrat), eine Phosphocellulosemembran, eine DEAE-Membran, eine Nylonnetzmembran oder eine Polytetrafluorethylenmembran handelt.

4. Verfahren nach einem der vorhergehenden Ansprüche, wobei die Membran (en) eine Porengröße von etwa 0,45 µm aufweist/aufweisen.

5. Verfahren nach einem der vorhergehenden Ansprüche, wobei das feste Kulturmedium ausgewählt ist aus der Gruppe bestehend aus FTM-A (flüssigem Thioglykolatmedium mit 1,075% Agar (Endkonzentration)), BHI (Brain-Heart-Infusion-Agar), anaerobem Difco-Brewer-Agar, R2A-Agar, Schaedler-Blutagar, Caso-Agar ICR (tryptischem Sojaagar), Columbia-Agar mit 5% Blut und anaerobem CDC-Blutagar.

6. Verfahren nach einem der vorhergehenden Ansprüche, wobei in (d) die Filtrierrückstandkulturen so lange kultiviert werden, bis eine nachweisbare Menge an Adenosintriphosphat produziert wurde.

7. Verfahren nach Anspruch 6, wobei die Filtrierrückstandkulturen etwa 2 bis etwa 7 Tage lang kultiviert werden.

8. Verfahren nach Anspruch 7, wobei die Filtrierrückstandkulturen 5 Tage lang kultiviert werden.

9. Verfahren nach einem der vorhergehenden Ansprüche, wobei in (e) eine lebensfähige Mikroorganismuszelle, Mikrokolonie oder Kolonie mit einem Lumineszenzassay nachgewiesen wird.

10. Verfahren nach Anspruch 9, wobei der Lumineszenzassay: (i) von einer lebensfähigen Mikroorganismuszelle, Mikrokolonie oder Kolonie auf der Membran produziertes Adenosintriphosphat nachweist; (ii)
einen Luciferaseassay umfasst; oder (iii) ein zwischen einer Sonde und einer einem Mikroorganismus endogenen Nukleinsäure gebildetes Nukleinsäurehybridisierungsprodukt nachweist, zum Beispiel mit einer Peroxidasereaktion.

11. Verfahren nach Anspruch 9 oder 10, wobei die Lumineszenz mit einer CCD-Kamera (CCD = Charged Coupled Device) und Software zur Bildanalyse nachgewiesen wird.

12. Verfahren nach einem der vorhergehenden Ansprüche, welches auf einem schnellen mikrobiologischen Nachweissystem durchgeführt wird, welches eine Station zur Probenvorbereitung, eine Autosprühstation, einen Nachweisturm, ein Gerät zur Bildanalyse, eine CCD-Kamera und einen Computer mit Software umfasst.

13. Verfahren nach einem der vorhergehenden Ansprüche, wobei es sich bei der pharmazeutischen Zusammensetzung um eine parenterale Zusammensetzung, eine orale Zusammensetzung, eine nasale Zusammensetzung oder eine okulare Zusammensetzung handelt.

14. Verfahren nach Anspruch 13, wobei es sich bei der pharmazeutischen Zusammensetzung um einen Impfstoff handelt, zum Beispiel ausgewählt aus der Gruppe bestehend aus Anthrax-Impfstoff, Tuberkulose-Impfstoff, Borreliose-Impfstoff, Diphterie-Toxoid- und Tetanus-Toxoid-Impfstoff, Diphterie-Toxoid- und Tetanus-Toxoid- und Pertussis-Impfstoff, Diphterie-Toxoid- und Tetanus-Toxoid- und azellulärem Pertussis-Impfstoff; Diphterie-Toxoid- und Tetanus-Toxoid- und azellulärem Pertussis- und *Haemophilius influenzae* Typ-b-Konjugat-Impfstoff, Diphterie-Toxoid- und Tetanus-Toxoid- und azelluärem Pertussis- und *Haemophilius influenzae* Typ-b-Konjugat- und inaktiviertem Poliovirus-Impfstoff, Hepatitis-A-Virus-Impfstoff, Hepatitis-A-Virus- und Hepatitis-B-Virus-Impfstoff, Hepatitis-B-Virus-Impfstoff, *Helicobacter pylori*-Impfstoff, *Haemophilus influenzae* Typ-b-Impfstoff, Influenza-Virus-Impfstoff, Poliovirus-Impfstoff, Meningokokken-(*Neisseria meningitidis*)-Impfstoff, Masernvirus-, Mumpsvirus-, Rötelnvirus-Impfstoff, Masernvirus-, Mumpsvirus-, Rötelnvirus- und Varicellavirus-Impfstoff, *Pneumokokken*-(*Streptococcus pneumoniae*)-Impfstoff, Tollwutimpfstoff, Respiratory-Syncytial-Virus-Impfstoff, Pockenimpfstoff, Toxoplasmose-(*Toxoplasma gondii*)-Impfstoff, Typhus-(*Salmonella typhi*)-Impfstoff, Tuberkulose-(*Mycobacterium tuberculosis*)-*Impfstoff* und Varicella-(Windpocken, Varicella-zoster-Virus)-Impfstoff; und wobei es sich bei dem Impfstoff vorzugsweise um einen Impfstoff gegen Vogelgrippe oder einen Impfstoff gegen Schweinegrippe handelt.

15. Verfahren zum Testen einer flüssigen pharmazeutischen Zusammensetzung auf Sterilität, bei dem man:
(a) eine filtrierbare pharmazeutische Zusammensetzung bereitstellt;
(b) die pharmazeutische Zusammensetzung filtriert, unter Bereitstellung von mindestens drei Filtermembranen, auf denen sich der Filtrierrückstand der pharmazeutischen Zusammensetzung abscheidet;
(c) die mindestens drei Filtermembranen auf feste Kulturmedien gibt und so mindestens drei Filtrierrückstandkulturen herstellt;
(d) (i) mindestens eine Filtrierrückstandkultur unter aeroben Bedingungen bei 20-25°C kultiviert; (ii) mindestens eine Filtrierrückstandkultur unter aeroben Bedingungen bei 30-35°C kultiviert und (iii) mindestens eine Filtrierrückstandkultur unter anaeroben Bedingungen bei 30-35°C kultiviert; mit der Maßgabe, dass keine der Filtrierrückstandkulturen länger als etwa 13 Tage lang kultiviert wird; und
(e) Adenosintriphosphat (ATP) auf der Membran nachweist, wobei das Vorhandensein von ATP auf einer Membran das Vorhandensein eines lebensfähigen Mikroorganismus in der pharmazeutischen Zusammensetzung anzeigt.

## Revendications

1. Procédé pour tester la stérilité d'une composition pharmaceutique liquide comprenant :
(a) la fourniture d'une composition pharmaceutique filtrable ;
(b) la filtration de la composition pharmaceutique pour obtenir au moins trois membranes filtrantes sur lesquelles le résidu de filtration de composition pharmaceutique est déposé ;
(c) le placement des au moins trois membranes filtrantes sur du milieu de culture solide pour produire au moins trois cultures de résidu de filtration ;
(d) la culture de (i) au moins une culture de résidu de filtration dans des conditions aérobies à 20-25 °C ; (ii) au moins une culture de résidu de filtration dans des conditions aérobies à 30-35 °C ; et (iii) au moins une culture de résidu de filtration dans des conditions anaérobies à 30-35 °C ; à condition qu'aucune des cultures de résidu de filtration ne soit cultivée pendant une durée supérieure à environ 13 jours ; et
(e) la détection d'une cellule, micro-colonie ou colonie de micro-organisme viable sur une membrane, la présence d'une cellule, micro-colonie ou colonie de micro-organisme viable sur la membrane indiquant la présence d'un micro-organisme viable dans la composition pharmaceutique.

2. Procédé de la revendication 1, dans lequel (b) comprend en outre la filtration d'une solution de lavage après la filtration de la composition pharmaceutique.

3. Procédé de la revendication 1 ou 2 dans lequel la/les membrane(s) est/sont une membrane de fluorure de polyvinylidène, une membrane de fibre de verre, une membrane de polycarbonate, une membrane de polyéthylène-téréphtalate, un ester de cellulose mixte (acétate de cellulose et nitrate de cellulose), une membrane de phosphocellulose, une membrane de DEAE, une membrane de toile de nylon, ou une membrane de polytétrafluoroéthylène.

4. Procédé de l'une quelconque des revendications précédentes, dans lequel la/les membrane(s) a/ont une taille de pore d'environ 0,45 µm.

5. Procédé de l'une quelconque des revendications précédentes, dans lequel le milieu de culture solide est choisi dans le groupe constitué de FTM-A (milieu de thioglycollate fluide contenant 1,075 % de gélose (concentration finale)), BHI (gélose de perfusion cerveau-coeur), gélose anaérobie de brasseur Difco, gélose R2A, gélose de Schaedler au sang, Caso-gélose ICR (gélose de soja trypsique), gélose Columbia à 5 % de sang, et gélose au sang anaérobie CDC.

6. Procédé de l'une quelconque des revendications précédentes, dans lequel, dans (d), les cultures de résidu de filtration sont cultivées pendant une durée suffisante pour la production d'une quantité détectable d'adénosine triphosphate.

7. Procédé de la revendication 6, dans lequel les cultures de résidu de filtration sont cultivées pendant une durée d'environ 2 à environ 7 jours.

8. Procédé de la revendication 7, dans lequel les cultures de résidu de filtration sont cultivées pendant une durée de 5 jours.

9. Procédé de l'une quelconque des revendications précédentes, dans lequel, dans (e), une cellule, micro-colonie ou colonie de micro-organisme viable est détectée en utilisant un dosage de luminescence.

10. Procédé de la revendication 9, dans lequel le dosage de luminescence : (i) détecte l'adénosine triphosphate produit par une cellule, micro-colonie ou colonie de micro-organisme viable sur la membrane ; (ii) comprend un dosage de luciférase ; ou (iii) détecte un produit d'hybridation d'acide nucléique formé entre une sonde et un acide nucléique endogène pour un micro-organisme, par exemple comprenant une réaction de peroxydase.

11. Procédé de la revendication 9 ou 10, dans lequel la luminescence est détectée en utilisant une caméra à dispositif coupleur de charge (CCD) et un logiciel d'analyse d'image.

12. Procédé de l'une quelconque des revendications précédentes, qui est conduit sur un système de détection de microbiologie rapide qui comprend une station de préparation d'échantillon, une station de pulvérisation automatique, une tour de détection, un analyseur d'image, une caméra CCD et un ordinateur avec un logiciel.

13. Procédé de l'une quelconque des revendications précédentes, dans lequel la composition pharmaceutique est une composition parentérale, une composition orale, une composition nasale, ou une composition oculaire.

14. Procédé de la revendication 13, dans lequel la composition pharmaceutique est un vaccin, par exemple, choisi dans le groupe constitué de vaccin contre l'anthrax ; vaccin contre la tuberculose ; vaccin contre la borréliose ; vaccin contre l'anatoxine d'hystérique et l'anatoxine tétanique ; vaccin contre l'anatoxine d'hystérique et l'anatoxine tétanique et la coqueluche ; vaccin contre l'anatoxine d'hystérique et l'anatoxine tétanique et la coqueluche acellulaire ; vaccin conjugué contre l'anatoxine d'hystérique et l'anatoxine tétanique et la coqueluche acellulaire et *Haemophilus influenzae* type b ; vaccin conjugué contre l'anatoxine d'hystérique et l'anatoxine tétanique et la coqueluche acellulaire et *Haemophilus influenzae* type b et le poliovirus inactivé ; vaccin contre le virus de l'hépatite A ; vaccin contre le virus de l'hépatite A et le virus de l'hépatite B ; vaccin contre le virus de l'hépatite B ; vaccin contre *Helicobacter pylori ;* vaccin contre *Haemophilus influenzae* type b ; vaccin contre le virus de la grippe ; vaccin contre le poliovirus ; vaccin contre les méningocoques (*Neisseria meningitidis*) ; vaccin contre le virus de la rougeole, le virus des oreillons, le virus de la rubéole ; vaccin contre le virus de la rougeole, le virus des oreillons, le virus de la rubéole et le virus de la varicelle ; vaccin contre les pneumocoques (*Streptococcus pneumoniae*) ; vaccin contre la rage ; vaccin contre le virus respiratoire syncytial ; vaccin contre la variole ; vaccin contre la toxoplasmose (*Toxoplasma gondii) ;* vaccin contre la typhoïde *(Salmonella typhi) ;* vaccin contre la tuberculose *(Mycobacterium tuberculosis) ;* et vaccin contre la varicelle (virus de la varicelle, varicelle-zona) ; et de préférence dans lequel ledit vaccin est un vaccin contre la grippe aviaire ou un vaccin contre la grippe porcine.

15. Procédé pour tester la stérilité d'une composition pharmaceutique liquide comprenant :
(a) la fourniture d'une composition pharmaceutique filtrable ;
(b) la filtration de la composition pharmaceutique pour obtenir au moins trois membranes sur lesquelles le résidu de filtration de composition pharmaceutique est déposé ;
(c) le placement des au moins trois filtres/membranes sur du milieu de culture solide pour produire au moins trois cultures de résidu de filtration ;
(d) la culture de (i) au moins une culture de résidu de filtration dans des conditions aérobies à 20-25 °C ; (ii) au moins une culture de résidu de filtration dans des conditions aérobies à 30-35 °C ; et (iii) au moins une culture de résidu de filtration dans des conditions anaérobies à 30-35 °C ; à condition qu'aucune des cultures de résidu de filtration ne soit cultivée pendant une durée supérieure à environ 13 jours ; et
(e) la détection d'adénosine triphosphate (ATP) sur la membrane, la présence d'ATP sur la membrane indiquant la présence d'un micro-organisme viable dans la composition pharmaceutique.
